(19) Europäisches Patentamt
European Patent Office
Office européen des brevets

(11) EP 2 345 889 A2

(12) EUROPÄISCHE PATENTANMELDUNG

(43) Veröffentlichungstag:
20.07.2011 Patentblatt 2011/29

(51) Int Cl.:
*G01N 21/64* (2006.01)     *G01J 3/44* (2006.01)

(21) Anmeldenummer: 11150974.1

(22) Anmeldetag: 14.01.2011

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 14.01.2010 DE 102010004774
09.04.2010 DE 202010004752 U

(71) Anmelder: Imm Photonics GmbH
85716 Unterschleissheim (DE)

(72) Erfinder:
• Raith, Friedrich
85716, Unterschleissheim (DE)
• Müller, Gerd
85716, Unterschleissheim (DE)

(74) Vertreter: Kramer - Barske - Schmidtchen
Landsberger Strasse 300
80687 München (DE)

(54) **Verfahren und Vorrichtung zum sicheren Emittieren einer Anregungsstrahlung sowie Sensorkopf für eine solche Vorrichtung**

(57) Eine Vorrichtung zum sicheren Emittieren einer Anregungsstrahlung (14) enthält eine Anregungsstrahlungsquelle (20) zum Erzeugen einer aus einem Anregungsstrahlungsaustrittsfenster austretenden Anregungsstrahlung (14), eine Referenzstrahlungsquelle (70) zum Erzeugen einer aus einem Referenzstrahlungsaustrittsfenster austretenden Referenzstrahlung (18) zur Beaufschlagung eines mit der Referenzstrahlung (18) und der Anregungsstrahlung (14) beaufschlagbaren Objekts (60), das sich an einer vorbestimmten Position befindet, mit der Referenzstrahlung (18), eine Detektoreinrichtung (30) zum Detektieren einer von dem Objekt (60) aufgrund der Beaufschlagung mit der Referenzstrahlung (18) erzeugten, durch ein Referenzrückstrahlungseintrittsfenster eintretenden Referenzrückstrahlung (15) und Erzeugen eines Detektionssignals basierend auf der detektierten Referenzrückstrahlung (15), eine Bestimmungseinrichtung (40) zum Überprüfen, ob das Detektionssignal wenigstens eine vorbestimmte Bedingung erfüllt, und eine Sicherheitseinrichtung (40) zum Ermöglichen einer Beaufschlagung des sich an der vorbestimmten Position befindenden Objekts (60) mit der Anregungsstrahlung (14) durch die Anregungsstrahlungsquelle (20) nur dann, wenn die vorbestimmte Bedingung erfüllt ist.

Fig. 2

EP 2 345 889 A2

**Beschreibung**

Die Erfindung betrifft ein Verfahren und eine Vorrichtung zum sicheren Emittieren einer Anregungsstrahlung, insbesondere ein Spektrometer mit integrierter Anregungsstrahlungsquelle. Zusätzlich betrifft die Erfindung einen Sensorkopf für eine solche Vorrichtung.

Herkömmliche Spektrometer sind meist als separate, mit einer externen Anregungsstrahlungsquelle verbundene Geräte ausgeführt.

Bei der Benutzung von Laserstrahlungsquellen zur Anregung ist es erforderlich, eine Aktivierung der Laserstrahlungsquelle nur dann zuzulassen, wenn sich der Strahlungsaustritt des Spektrometers in unmittelbarer Nähe des zu untersuchenden Objekts befindet.

Aus der DE 10 2006 032 701 B3 ist ein Gerät zum Detektieren einer laserangeregten Lumineszenzstrahlung bekannt, das ein mit einer Sensoröffnung ausgebildetes Gehäuse aufweist, wobei die Sensoröffnung für einen Austritt von von einem in dem Gehäuse enthaltenen Laser erzeugter Erregerstrahlung und einen Eintritt von durch die Erregerstrahlung angeregter, von einer zu untersuchenden Oberfläche ausgehender Lumineszenzstrahlung ausgebildet ist. Das Gehäuse enthält eine Einrichtung zum Überprüfen einer lichtdichten Berührung zwischen dem Rand der Sensoröffnung und der Oberfläche und eine Sicherheitsschaltung, die ein Einschalten des Lasers nur ermöglicht, wenn eine lichtdichte Berührung zwischen dem Rand der Sensoröffnung und der Oberfläche besteht.

Die US 2004/0109534 A1 beschreibt die Untersuchung eines Objekts mit Hilfe einer Fluoreszenzröntgenstrahlanalyse, bei der das Objekt mit einer Röntgenstrahlung als Anregungsstrahlung beaufschlagt wird und die von dem Objekt angestrahlte sekundäre Röntgenstrahlung analysiert wird. Damit eine optimale sekundäre Röntgenstrahlung entsteht, wird die primäre Röntgenstrahlung auf das Objekt fokussiert. Dazu wird der Abstand des Objekts von der Röntgenstrahlungsquelle, beispielsweise mittels einer Kamera, gemessen und der Fokus der Röntgenstrahlung dann entsprechend justiert.

Die DE 601 05 386 T2 beschreibt ein Verfahren zur Echtzeitbestimmung einer Festkörperzusammensetzung, bei dem auf einer Oberfläche des Festkörpers eine Glimmentladung erzeugt wird, die mittels optischer Emissionsspektroskopie analysiert wird. Gleichzeitig wird der Abstand zwischen der bestrahlten Fläche und einer festen Bezugsfläche mit Hilfe von Laserinterferometrie gemessen und die Tiefe der bestrahlten Fläche aus dem gemessenen Abstand ermittelt.

In der US 2006/0092415 A1 ist ein Verfahren zur Durchführung einer laserspektroskopischen Untersuchung einer Probe beschrieben, bei dem ein gepulster Laserstrahl automatisch auf ein Objekt fokussiert wird, um ein laserinduziertes Plasma zu erzeugen, das ausgewertet wird. Vor der Plasmaerzeugung wird der Abstand der Oberfläche des Objekts von der Autofokuseinrichtung gemessen und werden zusätzlich geometrische Parameter einer potentiellen Messstelle auf der Oberfläche des Objekts bestimmt. Die Analyse der von dem Plasma ausgesandten Strahlung erfolgt nur für solche potentiellen Messstellen, bei denen wenigstens ein zusätzlicher Parameter innerhalb eines vorbestimmten Toleranzbereiches liegt.

Aufgabe der Erfindung ist es, eine kostengünstige Möglichkeit zu schaffen, mit der eine Anregungsstrahlung, wie eine Laserstrahlung, nur bei Vorliegen bestimmter Sicherheitsbedingungen emittiert wird.

Diese Aufgabe wird mit einer Vorrichtung nach Anspruch 1 und einem Verfahren nach Anspruch 15 gelöst.

Die Unteransprüche sind auf vorteilhafte Ausführungsformen und Weiterbildungen der erfindungsgemäßen Vorrichtung gerichtet.

Der Anspruch 14 kennzeichnet einen erfindungsgemäßen, für eine erfindungsgemäße Vorrichtung vorgesehenen Sensorkopf.

Die Erfindung wird im Folgenden anhand von Figuren beispielhaft und mit weiteren Einzelheiten erläutert.

Es zeigen:

Fig. 1        eine vereinfachte schematische Ansicht eines Spektrometers,

Fig. 2        eine vereinfachte schematische Ansicht einer Vorrichtung gemäß einer ersten Ausführungsform der Erfindung,

Fig. 3        eine vereinfachte schematische Ansicht einer Vorrichtung gemäß einer zweiten Ausführungsform der Erfindung,

Fig. 4        eine vereinfachte schematische Ansicht einer Vorrichtung gemäß einer dritten Ausfiihrungsform der Erfindung,

Fig. 5        eine vereinfachte schematische Ansicht einer Vorrichtung gemäß einer vierten Ausführungsform der Erfindung,

Fig. 6        eine vereinfachte schematische Ansicht einer Vorrichtung gemäß einer fünften Ausführungsform der Erfindung,

Fig. 7        eine vereinfachte perspektivische Ansicht der Ausführungsform aus Fig. 6,

Fig. 8        eine schematische Darstellung einer Kopplungseinrichtung für die Ausführungsform aus Fig. 6,

Fig. 9        eine schematische Darstellung einer alternativen Kopplungseinrichtung für die Ausführungsform aus Fig. 6,

Fig. 10    eine beispielhafte Anordnung eines Strahlungsaustritts- und eines Strahlungseintrittsfensters und einer Objektoberfläche,

Fig. 11    sich bei der Anordnung gemäß Fig. 10 ergebende Intensitätskurven,

Fig. 12    eine gegenüber Fig. 10 abgeänderte Anordnung von Strahlungsaustritts- und Strahlungseintrittsfenster,

Fig. 13    sich bei der Anordnung gemäß Fig. 12 ergebende Intensitätskurven,

Fig. 14    eine weitere Anordnung von Strahlungsaustritts- und Strahlungseintrittsfenstern,

Fig. 15 5    eine perspektivische Ansicht eines Sensorkopfes, und

Fig. 16    eine perspektivische Ansicht eines autarken Gerätes mit teilweise entferntem Gehäuse.

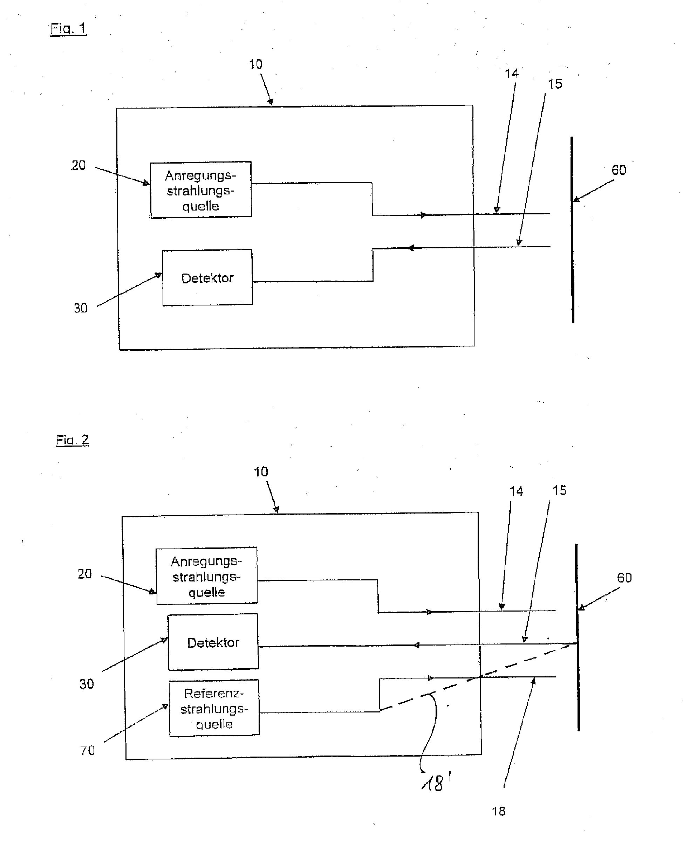

Gemäß Fig. 1 enthält ein beispielsweise aus der DE 10 2006 032 701 B3 bekanntes Spektrometer ein Gehäuse 10 mit einer (nicht gezeigten) rechtsseitigen Gehäuseöffnung. In dem Gehäuse 10, das beispielsweise aus Metall oder Kunststoff besteht, befinden sich eine Anregungsstrahlungsquelle 20 für eine Anregungsstrahlung 14, wie eine Laserstrahlung, und ein Detektor 30. Die von der Anregungsstrahlungsquelle 20 emittierte Strahlung wird auf bekannte Weise über eine geeignete Optik zu der Gehäuseöffnung geleitet und tritt über ein Anregungsstrahlungsaustrittsfenster, beispielsweise über einen ersten Lichtleiter, wie eine Faser, in Richtung eines vor der Gehäuseöffnung positionierten Objekts 60 aus und regt das Objekt 60 an, beispielsweise über eine Zwei-Photonen-Anregung oder eine Lumineszenzanregung. Über einen zweiten, auf das Objekt 60 gerichteten Lichtleiter tritt eine aufgrund der Anregung von dem Objekt 60 ausgesandte Anregungsrückstrahlung 15 über ein Anregungsstrahlungseintrittsfenster durch die Gehäuseöffnung in das Gehäuse 10 ein und wird über eine geeignete Optik zu einem Detektor 30 geleitet. Der Detektor 30 detektiert die Anregungsrückstrahlung 15 und erzeugt basierend darauf ein Signal, das für eine Weiterverarbeitung zu einer (nicht gezeigten) Datenverarbeitungseinrichtung übertragen wird.

Da der eigentliche Messvorgang, d.h. die Auswertung der Anregungsrückstrahlung, nicht Bestandteil der vorliegenden Erfindung sind, wird er hier nicht im Einzelnen erläutert.

Gemäß Fig. 2 weist eine erste Ausführungsform der Erfindung zusätzlich zu der in Fig. 1 gezeigten Anordnung in dem Gehäuse 10 eine bei direkter Betrachtung für das Auge unschädliche Referenzstrahlungsquelle 70 wie z.B. eine Lampe, eine LED, eine SLED oder eine Laserlichtquelle auf. Die von der Referenzstrahlungsquelle 70 erzeugte Referenzstrahlung 18 wird über eine geeignete Optik zu der Gehäuseöffnung geleitet und tritt über ein Referenzstrahlungsaustrittsfenster, beispielsweise über einen dritten Lichtleiter, in Richtung des vor der Gehäuseöffnung positionierten Objekts 60 aus. Entsprechend geht von dem Objekt 60 eine Referenzrückstrahlung 15 aus, die beispielsweise durch Reflexion der Referenzstrahlung 18 an dem Objekt 60 erzeugt wird. Die Referenzrückstrahlung 15 tritt über ein Referenzrückstrahlungseintrittsfenster in den zweiten Lichtleiter ein und wird zu dem Detektor 30 geleitet. Der Detektor 30 detektiert die Referenzrückstrahlung und erzeugt basierend darauf ein Detektionssignal, das beispielsweise Informationen über das Spektrum der Referenzrückstrahlung wie eine Wellenlängenverteilung, eine Intensität bei verschiedenen Wellenlängen, eine Peak-Wellenlänge, eine Peak-Breite und eine Peak-Intensität bzw. Peak-Amplitude enthält.

Das Detektionssignal kann zu einer Bestimmungseinrichtung, wie einer Datenverarbeitungseinrichtung, weitergegeben werden, die die in dem Detektionssignal enthaltenen spektralen Informationen mit in einem der Referenzstrahlungsquelle 70 zugeordneten Referenzsignal enthaltenen spektralen Informationen vergleicht, beispielsweise, indem die Peak-Wellenlängen und die Peak-Intensitäten der beiden Spektren verglichen werden. Die Bestimmungseinrichtung kann in dem Gehäuse 10 angeordnet sein, oder sie kann sich außerhalb des Gehäuses 10 befinden und über einen in dem Gehäuse 10 ausgebildeten Anschluss mit dem Detektor 30 verbunden sein. Das Referenzsignal kann für die Referenzstrahlungsquelle im Voraus erzeugt worden sein, beispielsweise durch Aufnehmen eines Referenzspektrums bei bekanntem Abstand eines Objekts zu der Gehäuseöffnung, und kann in einem Speicher der Bestimmungseinrichtung gespeichert sein.

Bei einer Übereinstimmung der Peak-Wellenlänge, beispielsweise, wenn eine Differenz der beiden Peak-Wellenlängen kleiner als ein bestimmter Schwellenwert ist, und für den Fall, dass die Peak-Intensität des Detektionssignals einen vorbestimmten Schwellenwert überschreitet, bestimmt die Bestimmungseinrichtung, dass der Abstand zwischen dem Objekt 60 und der Gehäuseöffnung unter einem vorgegebenen Mindestabstand liegt und die sichere Emission der Anregungsstrahlung gewährleistet ist, d.h. beispielsweise, dass Bedingungen vorliegen, unter denen keine Anregungsstrahlung in das Auge eines Betrachters gelangen kann. Andernfalls, wenn beispielsweise die Peak-Wellenlängen zu weit auseinander liegen oder die Peak-Intensität des Detektionssignals zu niedrig ist, bestimmt die Bestimmungseinrichtung, dass es wegen eines zu großen Objektabstandes nicht sicher ist, die Anregungsstrahlung zu emittieren. In diesem Fall gibt die Bestimmungseinrichtung ein Signal zu einer als eine Sicherheitseinrichtung dienenden Datenverarbeitungsschaltung aus, das bewirkt, dass die Sicherheitseinrichtung eine Aktivierung der Anregungsstrahlungsquelle verhindert. Die Sicherheitseinrichtung kann ebenfalls innerhalb oder außerhalb des Gehäuses 10 angeordnet sein.

Zur Sicherstellung, dass der Abstand zwischen dem Objekt 60 und der Öffnung des Gehäuses 10 unter einem vorgegebenen Mindestabstand liegt, gibt es unterschiedlichste Verfahren. Die Intensität der Referenzrückstrahlung 15 nimmt von sehr kleinen Werten bei einem sehr kleinen Abstand zwischen dem Objekt 60 und der Gehäuseöffnung mit zunehmendem Abstand zu und nimmt nach Durchlaufen eines Maximums bei weiter größer werdendem Abstand wieder ab. Ein Schwellwert der Intensität der Referenzrückstrahlung kann derart festgelegt werden, dass bei seinem Überschreiten das Unterschreiten eines Mindestabstandes gewährleistet ist. Dieser Schwellwert kann für zahlreiche Objektoberflächen unabhängig von der Art der jeweiligen Objektoberfläche festgelegt werden. Für eine genauere Festlegung kann der Schwellwert abhängig von der Art des Objektes, insbesondere vom Material von dessen Oberfläche, festgelegt werden. Eine Kurve, die die Intensität der Referenzrückstrahlung in Abhängigkeit vom Abstand zwischen dem Objekt und der Gehäuseöffnung angibt, hängt vom Abstand zwischen der Austrittsöffnung der Referenzstrahlung aus dem Gehäuse 10 und der Eintrittsöffnung für die Referenzrückstrahlung, dem Winkel zwischen der Referenzstrahlung und der vom Detektor ausgewerteten Referenzrückstrahlung sowie der Oberfläche des Objekts beziehungsweise dessen Reflektivität ab. Die Form der Kurve, insbesondere der Abstand zwischen Objekt und Gehäuse, bei dem die Reflektionsrückstrahlung ihr Maximum erreicht, ist jedoch von der Art der Oberfläche des Gegenstandes weitgehend unabhängig. Somit kann durch die Bestimmung des Maximums der Reflektionsrückstrahlung und Arbeiten mit einem Abstand, bei dem die Reflektionsrückstrahlung ihr Maximum hat, weitgehend unabhängig von der Art des Objektes sichergestellt werden, dass ein vorbestimmter Mindestabstand eingehalten wird.

Bezug nehmend wiederum auf Figur 2, haben die am Gehäuse 10 ausgebildete Austrittsöffnung, aus der die Referenzstrahlung 18 in Richtung auf das Objekt 60 austritt, und die am Gehäuse 10 ausgebildete Eintrittsöffnung, durch die vom Objekt 60 abgestrahlte Referenzrückstrahlung 15 in das Gehäuse für eine Auswertung durch den Detektor 30 eintritt, einen vorbestimmten Abstand voneinander. Weiter verlaufen die in Figur 2 schematisch durch gerade Linien dargestellte Mittellinien der Referenzstrahlung 18 und der Referenzrückstrahlung 15 nicht parallel zueinander, sondern schneiden sich in einem vorbestimmten Abstand von dem Gehäuse 10. Bevorzugt befindet sich das Objekt 60 im Schnittpunkt der Geraden 18 (gestrichelt eingezeichnet), die beispielsweise die axiale Richtung einer Lichtleiterfaser, durch deren Austrittsöffnung die Referenzstrahlung 18 austritt, verlängert, mit der Verlängerung der axialen Richtung eines Lichtleiters, durch dessen Eintrittsöffnung die Anregungsrückstrahlung 15 in das Gehäuse 10 eintritt. Bei Anordnung der Oberfläche des Objekts 16 im Schnittpunkt der beiden Geraden ist die vom Detektor 30 aufgenommene Intensität der Referenzrückstrahlung im Allgemeinen größer als bei einem Abstand zwischen dem Objekt 60 und dem Gehäuse 10, der größer oder kleiner als der Abstand des Schnittpunktes der zueinander geneigten Geraden 15 und 18' vom Gehäuse 10 ist. Ähnlich ist die Richtung der auf das Objekt 60 treffenden Anregungsstrahlung 14 vorteilhaft zur Rückstrahlung 15 hin geneigt, wodurch die Intensität der vom Detektor 30 erfassten Anregungsrückstrahlung erhöht wird.

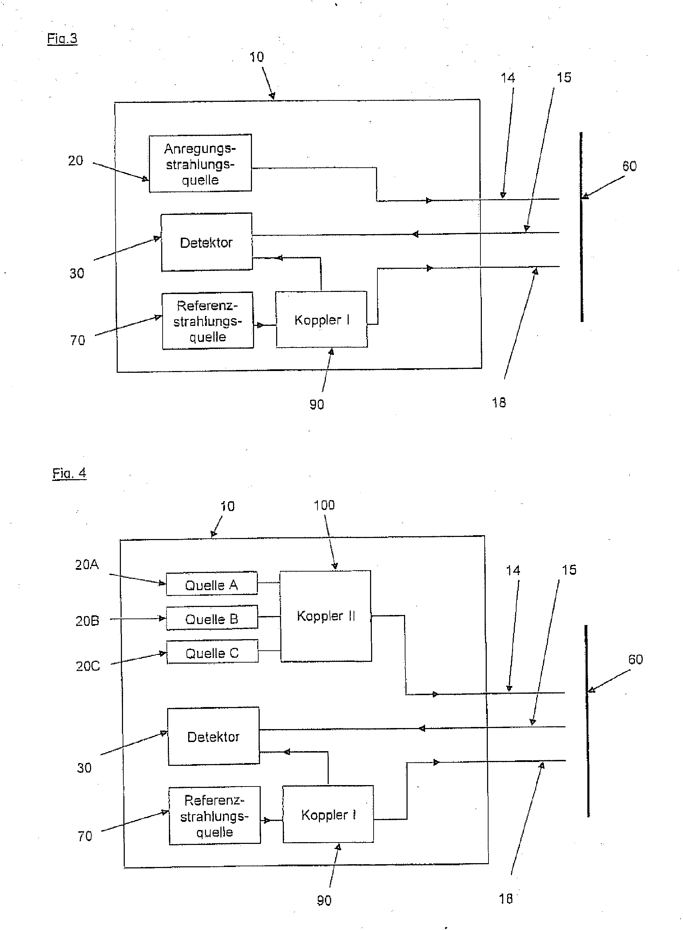

Bei einer in Fig. 3 gezeigten zweiten Ausführungsform der Erfindung ist weiter eine erste Kopplungseinrichtung 90 in dem Gehäuse 10 angeordnet, die beispielsweise über eine Spiegelanordnung mit einem in dem Strahlengang der Referenzstrahlung 18 angeordneten teilweise durchlässigen Spiegel einen Teil der Referenzstrahlung 18 zu dem Detektor 30 leitet. Der Detektor 30 kann basierend auf der Referenzstrahlung 18 das für den Vergleich mit dem Detektionssignal benötigte Referenzsignal erzeugen.

Bei einer in Fig. 4 gezeigten dritten Ausführungsform der Erfindung sind in dem Gehäuse 10 mehrere, z.B. drei, unterschiedliche Anregungsstrahlungsquellen 20A, 20B und 20C angeordnet, die über eine zweite Kopplungseinrichtung 100 miteinander gekoppelt werden, beispielsweise durch Leiten der von den unterschiedlichen Anregungsstrahlungsquellen emittierten Anregungsstrahlung zu dem ersten Lichtleiter über eine geeignete Anordnung von Spiegeln.

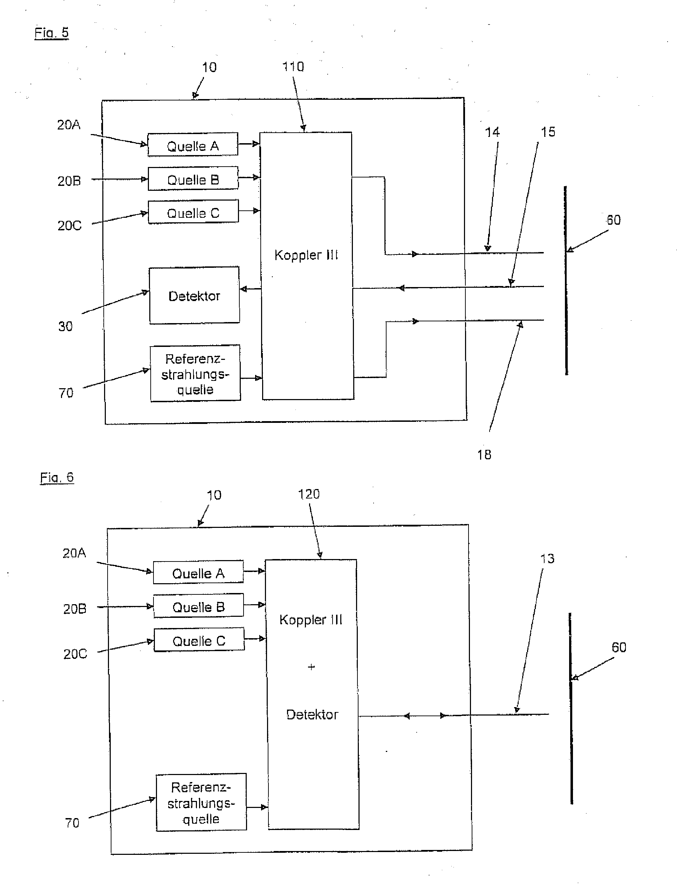

Bei einer in Fig. 5 gezeigten vierten Ausführungsform der Erfindung sind die beiden Kopplungseinrichtungen 90 und 100 in einer dritten Kopplungseinrichtung 110 integriert.

Bei den Ausführungsformen gemäß Fig. 3 bis 5 sind die die Strahlungen 14, 15 und 18 darstellenden Geraden vorteilhafterweise ähnlich zueinander geneigt wie anhand Fig. 2 beschrieben. Bei einer in Fig. 6 gezeigten fünften Ausführungsform der Erfindung ist der Detektor 30 in die dritte Kopplungseinrichtung 110 integriert, und die integrierte Kopplungseinrichtung 120 ist derart ausgebildet, dass die Anregungsstrahlung, die Referenzstrahlung, die Referenzrückstrahlung und die Anregungsrückstrahlung über einen gemeinsamen Strahlungsweg 13 z.B. über eine Lichtleiterfaser aus dem Gehäuse aus- bzw. in das Gehäuse eintreten. Bei dieser Ausführungsform wird die im Strahlungsweg 13 enthaltene Referenzrückstrahlung beispielsweise lediglich dahingehend ausgewertet, dass sie bezüglich ihrer Wellenlänge in vorbestimmter Weise mit der Referenzstrahlung übereinstimmt und in einer gewissen Intensität vorhanden ist. Damit wird, wenn das Objekt 60 beispielsweise eine in einen Schlitz einzuführende Karte ist, gewährleistet, dass sich im Schlitz ein Objekt 60 befindet. Für den Fall, dass sich im Schlitz kein Objekt befindet, kann Anregungslicht in gefährlicher Weise nach außen gelangen, ohne von dem Objekt 60 reflektiert zu werden.

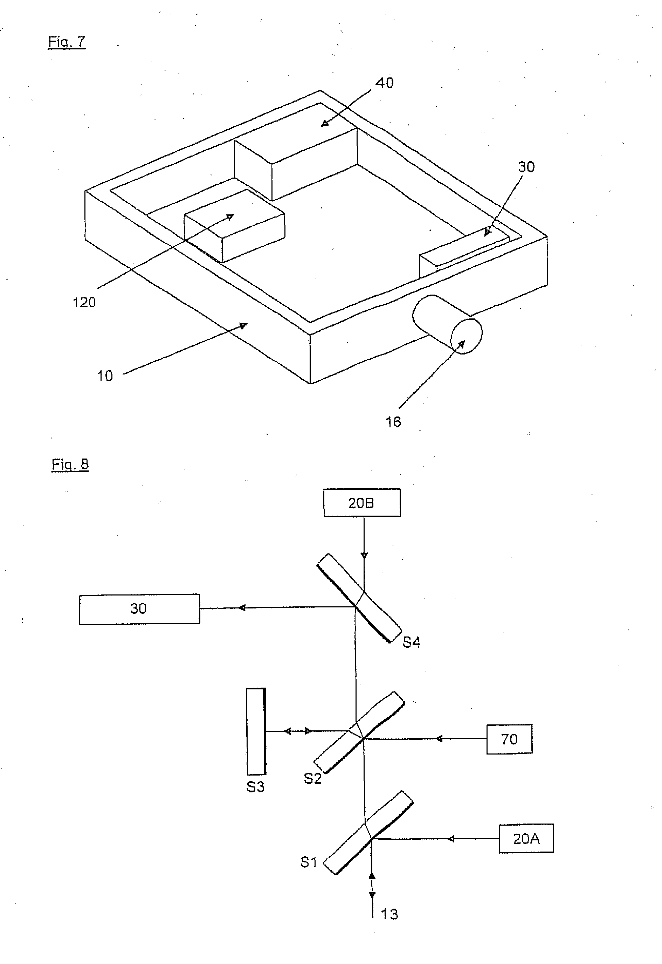

Fig. 7 zeigt eine vereinfachte perspektivische Ansicht der Ausführungsform aus Fig. 6. Die in Fig. 7 gezeigte Vorrichtung ist als ein autarkes Handgerät ausgebildet, in das die Bestimmungseinrichtung und die Sicherheitseinrichtung in Form einer Datenverarbeitungseinrichtung 40 integriert ist. Die von der Kopplungseinrichtung 120 ausgehende Strahlung tritt über eine in der Gehäuseöffnung angebrachte Lichtleiterfaser 16 aus dem Gehäuse aus, über die die Objektstrahlung in das Gehäuse eintreten kann, wo sie dann zu dem Detektor 30 geleitet wird. In dem Gehäuse 10 ist eine nicht gezeigte Stromversorgung zur Energieversorgung der Strahlungsquellen, des Detektors 30 und der Datenverarbeitungseinrichtung 40 enthalten. Die Datenverarbeitungseinrichtung 40 kann eine Auswerteeinrichtung für die von dem Detektor detektierte Anregungsobjektstrahlung enthalten, und in dem Gehäuse kann eine Anzeigeeinheit zum Anzeigen des Resultats der Auswertung durch die Auswerteeinrichtung enthalten sein. Der Begriff Datenverarbeitungseinrichtung wird im Vorstehenden sehr breit verstanden. Beispielsweise kann die Datenverarbeitungseinrichtung 40 ein Gitter, ein Prisma oder eine sonstige Einrichtung enthalten, mit der Licht wellenlängenabhängig zerlegt werden kann. Dieser passiv arbeitenden Einrichtung kann ein lichtempfindliches Array nachgeschaltet sein, auf das die hinsichtlich ihrer Wellenlängen zerlegte Strahlung auftrifft, wobei dem Array ein Rechner nachgeschaltet sein kann, der die wellenlängenabhängigen Anteile der Strahlung auswertet.

Wahlweise kann die in Fig. 7 gezeigte Ausführungsform jedoch auch als eine nicht autarke Einrichtung ausgebildet sein, bei der beispielsweise die Stromversorgung und/oder die Auswerteeinrichtung und/oder die Anzeigeeinheit außerhalb des Gehäuses angeordnet sind. In diesem Fall weist das Gehäuse mindestens einen Anschluss zur Verbindung mit einer externen Stromversorgung und/oder einem externen Rechner auf. Denkbar ist auch, dass die Datenverarbeitungseinrichtung 40 extern ist.

Fig. 8 zeigt eine schematische Darstellung einer beispielhaften Ausführung der Kopplungseinrichtung 120 für den Fall, dass zwei Anregungsstrahlungsquellen 20A und 20B vorgesehen sind, wobei für die Wellenlängen beispielsweise gilt, dass die Wellenlänge der von der Anregungsquelle 20A emittierten Anregungsstrahlung kleiner als die Wellenlänge der Referenzstrahlung ist, die wiederum kleiner als die Wellenlänge der von der Anregungsquelle 20B emittierten Anregungsstrahlung ist. Die von der Referenzstrahlungsquelle 70 ausgesandte Referenzstrahlung trifft auf einen für die Referenzstrahlung teilweise durchlässigen dichroitischen ersten Spiegel S2, wobei ein erster Teil der Referenzstrahlung in Richtung eines für die Referenzstrahlung durchlässigen dichroitischen zweiten Spiegels S1 reflektiert und ein zweiter Teil der Referenzstrahlung in Richtung eines die Referenzstrahlung zurück zu dem ersten Spiegel S2 reflektierenden dritten Spiegels S3 transmittiert wird. Die zurückreflektierte Referenzstrahlung wird an dem ersten Spiegel S2 in Richtung eines die Referenzstrahlung reflektierenden dichroitischen vierten Spiegels S4 reflektiert, an dem sie zur Erzeugung des Referenzsignals in Richtung des Detektors 30 reflektiert wird.

Der erste Teil der Referenzstrahlung tritt über den Spiegel S1 auf dem Strahlungsweg 13 aus dem Gehäuse 10 aus und beaufschlagt das vor der Gehäuseöffnung angeordnete Objekt 60. Die Referenzobjektstrahlung tritt auf dem Strahlungsweg 13 in das Gehäuse ein und gelangt über die Spiegel S1, S2 und S4 zu dem Detektor 30 zur Erzeugung des Detektionssignals.

Die von der ersten Anregungsstrahlungsquelle 20A emittierte Anregungsstrahlung wird an dem vorzugsweise für dieselbe undurchlässigen zweiten Spiegel S1 in Richtung der Gehäuseöffnung reflektiert, tritt auf dem Strahlungsweg 13 aus dem Gehäuse 10 aus und beaufschlagt das Objekt 60. Die von der zweiten Anregungsstrahlungsquelle 20B erzeugte Anregungsstrahlung tritt auf geradem Wege durch die für dieselbe zumindest teilweise durchlässigen Spiegel S4, S2 und S1 auf dem Strahlungsweg 13 aus dem Gehäuse 10 aus und beaufschlagt das Objekt 60. Die von dem Objekt 60 ansprechend auf die Anregungsstrahlung erzeugte Anregungsobjektstrahlung tritt auf dem Strahlungsweg 13 in das Gehäuse 10 ein und gelangt über die Spiegel S1, S2 und S4 zu dem Detektor 30, wobei der zweite Spiegel S1 und der erste Spiegel S2 für die Anregungsobjektstrahlung zumindest teilweise durchlässig sind und der vierte Spiegel S4 für dieselbe möglichst gut reflektierend ist.

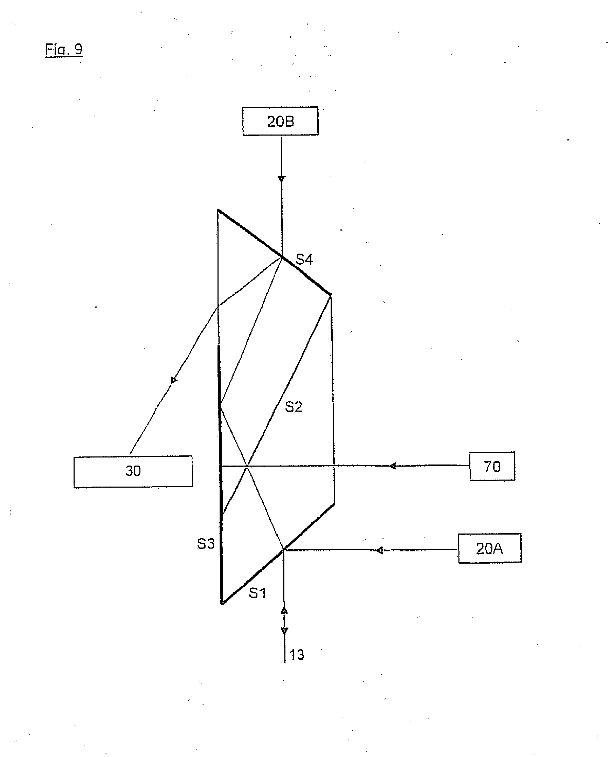

Fig. 9 zeigt eine andere beispielhafte Ausführung der Kopplungseinrichtung 120, die aus Prismen zusammengesetzt ist. Die Funktionsweise der in Fig. 9 gezeigten Ausführung ist analog zu der unter Bezugnahme auf Fig. 8 beschriebenen Funktionsweise, wobei Grenzflächen der Prismen die Funktion der (teilweise durchlässigen) Spiegel S1 bis S4 haben. Von einer detaillierten Beschreibung wird daher abgesehen.

Bei den vorstehend beschriebenen Ausführungsformen kann die Sicherheit vor einer unbeabsichtigten Emission der Anregungsstrahlung dadurch erhöht werden, dass, wenn die Referenzstrahlung 18 eine erste Polarisation aufweist, in dem Gehäuse 10 auf dem Strahlungsweg der Referenzobjektstrahlung 15 ein Filter, beispielsweise ein Gitter oder eine auf einen Spiegel aufgebrachte Polarisator- oder Analysatorschicht, angeordnet ist, das die Referenzobjektstrahlung mit der ersten Polarisation herausfiltert. Auf diese Weise gelangt bei einer Reflexion der Referenzstrahlung an einem für die Referenzstrahlung transparenten Objekt keine Referenzobjektstrahlung zu dem Detektor 30 und die Sicherheitseinrichtung verhindert die Aktivierung der Anregungsstrahlungsquelle.

Die Strahlung muss nicht über eine gemeinsame Lichtleiterfaser aus dem Gehäuse austreten oder in dasselbe eintreten. Es könnten mehrere Lichtleiterfasern für die jeweiligen Strahlungsarten vorgesehen sein, die durch separate oder gemeinsame Öffnungen in dem Gehäuse geführt sein können. Alternativ kann auch keine Lichtleiterfaser vorgesehen sein, und die Strahlung kann direkt durch die Gehäuseöffnung austreten, die beispielsweise von einem für die jeweiligen Strahlungsarten transparentem Fenster verschlossen sein kann.

An dem Gehäuse 10 können verschiedene Bedienelemente wie Schalter etc. vorgesehen sein, beispielsweise zur Aktivierung der Anregungsstrahlungsquelle, wobei diese Aktivierung mit der Aktivierung der Referenzstrahlungsquelle verbunden ist und wie vorher beschrieben nur dann erlaubt wird, wenn die sichere Emission der Anregungsstrahlung gewährleistet ist.

Die erfindungsgemäße Vorrichtung kann in unterschiedlichster Weise ausgebildet sein. Beispielsweise kann sie in Form eines autarken kompakten Gerätes ausgebildet sein, das mit einer Hand handhabbar ist und alle beschriebenen Bauteile einschließlich einer Energieversorgung enthält. Alternativ oder zusätzlich können Auswerteeinrichtungen und/oder Energieversorgung an entsprechende Anschlüsse des Gerätes anschließbar sein. Als Laser können Diodenlaser VCSEL (vertical cavity surface emitting laser diode), Super-LEDs, einfach LEDs oder sonst welche Strahlungsquellen verwendet werden, die im Infrarotbereich, sichtbaren Wellenlängenbereich oder UV-Bereich arbeiten.

Das Gerät wird auf das zu prüfende Objekt zu und/oder von ihm weg bewegt, bis die Auswerteeinrichtung ermittelt und anzeigt, dass bezüglich der Referenzrückstrahlung die vorstehend geschilderten vorbestimmten Bedingungen vorliegen. Unter möglichster Beibehaltung des Abstandes zwischen dem Messkopf und dem Objekt wird das Objekt dann mit Anregungsstrahlung beaufschlagt, wobei die Anregungslichtquelle abgeschaltet wird sobald zumindest eine von vorbestimmten Bedingungen nicht mehr erfüllt ist. Die Bewegung des Gerätes relativ zum Objekt kann von Hand oder mittels eines Aktors erfolgen.

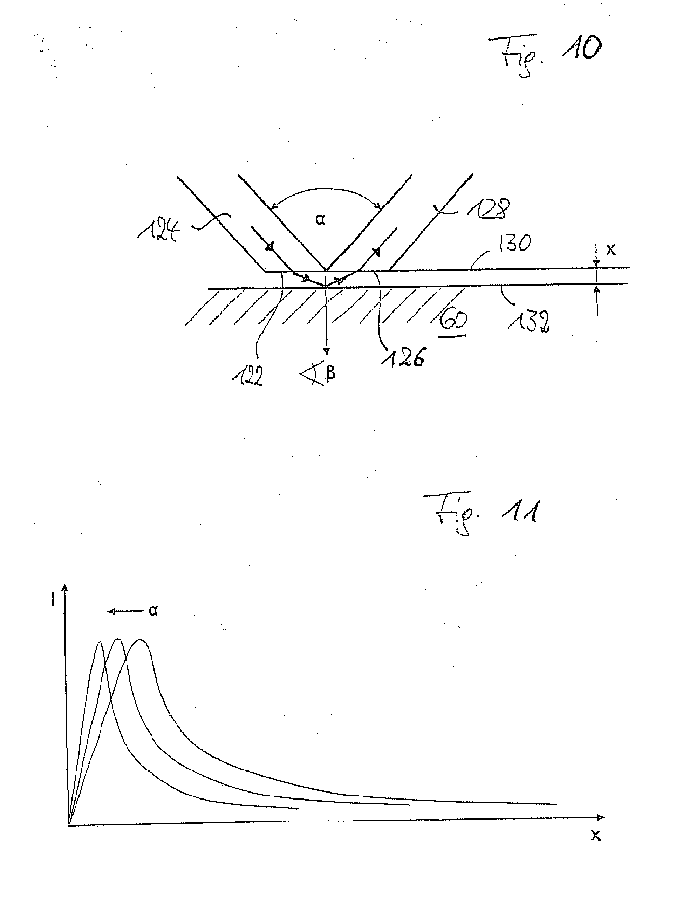

Anhand der Fig. 10, 12 und 14 werden im Folgenden Beispiele für die Anordnung von Lichtleitern mit Strahlungsaustrittsfenstern und Strahlungseintrittsfenstern erläutert.

Gemäß Fig. 10 ist ein Strahlungsaustrittsfenster 122 eines Lichtleiters 124 unmittelbar benachbart einem Strahlungseintrittsfenster 126 eines weiteren Lichtleiters 128 angeordnet. Die Längsrichtungen der Lichtleiter bilden einen Winkel $\alpha$. Die Enden der Lichtleiter 124 und 128 sind jeweils schräg zu deren Längsrichtungen geschnitten, so dass sie die in einer gemeinsamen Ebene liegenden Strahlungsaustritts- bzw. Strahlungseintrittsfenster bilden. In einem Abstand x von der gemeinsamen Ebene 130 der Fenster 122 und 126 ist die Oberfläche 132 des Objekts 60 angeordnet.

Ein aus der Faser bzw. dem Lichtleiter 124 austretender Lichtstrahl trifft unter dem Winkel $\alpha/2$ auf das Strahlungsaustrittsfenster 122 und tritt gemäß dem Brechungsgesetz unter dem Winkel $\beta/2$ aus dem Austrittsfenster 122 aus. Dadurch vergrößert sich der effektive Winkel zwischen dem auf die Oberfläche 132 auftreffenden und in den Lichtleiter 128 rückreflektierten Strahl auf den Wert:

$$\beta = 180° - 2 \text{ x } (\arcsin(n) \text{ x } \sin(\alpha/2))$$

wobei n der Brechungsindex des Lichtleiters ist.

Vorteilhaft wird x so gewählt, dass sich der aus dem Lichtleiter 124 austretende Lichtstrahl und der in den Lichtleiter 128 eintretende Lichtstrahl auf der Oberfläche 132 schneiden.

Fig. 11 gibt für verschiedene Werte von $\alpha$ die Intensität I als die Lichtmenge an, die bei konstanter aus dem Lichtleiter 124 austretender Lichtmenge in den Lichtleiter 128 eintritt. Wie ersichtlich, ergibt sich für jeden Wert von $\alpha$ eine Kurve, die mit zunehmendem Abstand x zunächst etwa linear ansteigt und nach Durchlaufen eines Maximums etwa exponentiell abnimmt. Dabei sind die Maxima etwa gleich hoch und verschieben sich mit zunehmendem $\alpha$ zu kleineren Werten von x.

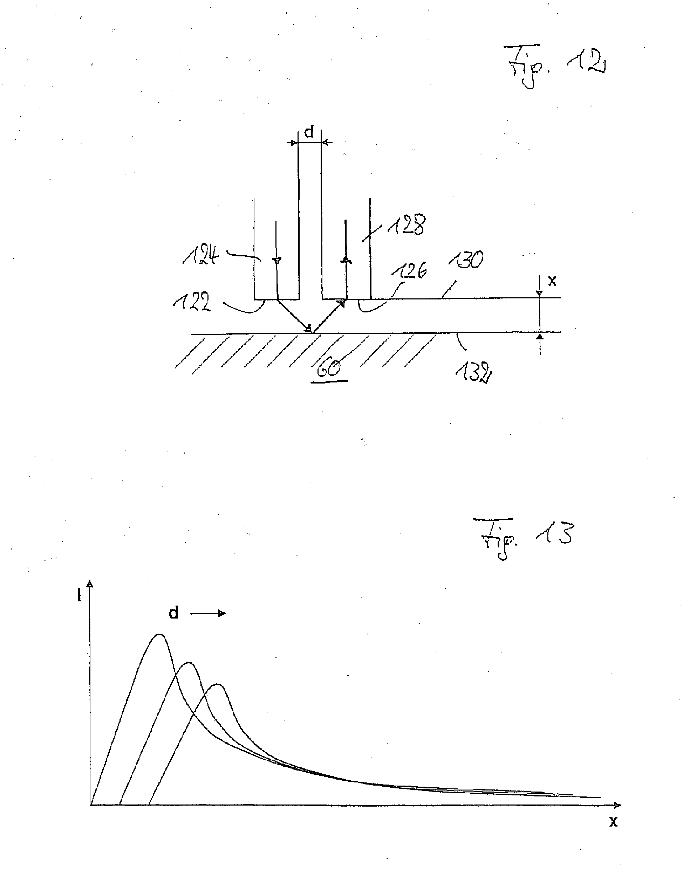

Fig. 12 verdeutlicht die Verhältnisse für einen Fall, bei dem die Lichtleiter 124, 128 in einem gegenseitigen Abstand d angeordnet sind und die Lichtleiter an ihren Endflächen senkrecht zur Längserstreckung geschnitten sind, so dass das Austrittsfenster 122 und das Eintrittsfenster 126 jeweils senkrecht zur Längsrichtung des zugehörigen Lichtleiters in der gemeinsamen Ebene 130 angeordnet sind.

Fig. 13 gibt die Intensitätsverhältnisse für die Anordnung gemäß Fig. 12 wieder. Wie ersichtlich, steigt die Intensität der in den Lichtleiter 128 einfallenden Strahlung zunächst linear mit dem Abstand an und fällt nach Durchlaufen eines Maximums annähernd exponentiell ab, wobei die Höhe des Maximums mit zunehmenden Abstand d zwischen den Lichtleitern abnimmt und sich die Lage des Maximums mit zunehmenden Abstand d zu größer werdendem Abstand x schiebt.

Vorteilhafterweise befindet sich beim Beaufschlagen der Oberfläche 132 des Objekts 60 die Oberfläche 132 in einem Abstand x von der Fensterebene 130, der dem Maximum der Fig. 11 und 13 entspricht. Durch geeignete Wahl des Winkels $\alpha$ und der Distanz d zusammen mit entsprechenden Parametern des jeweiligen Lichtleiters, wie Material, Durchmesser, numerische Apparatur, usw., kann der gewünschte Arbeitsabstand eingestellt werden. Dabei ist nicht in allen Fällen das Arbeiten im Maximum der jeweiligen Intensitätskurve vorteilhaft. Aus beispielsweise Tiefenschärfeforderungen kann es vorteilhaft sein, mit einem Arbeitsabstand zu arbeiten, der auf der steigenden oder fallenden Flanke der jeweiligen Intensitätskurve liegt. Beispielsweise kann es aus regelungstechnischen Gründen vorteilhaft sein, den Arbeitsabstand in die Mitte des linearen Bereiches der ansteigenden Flanke zu legen.

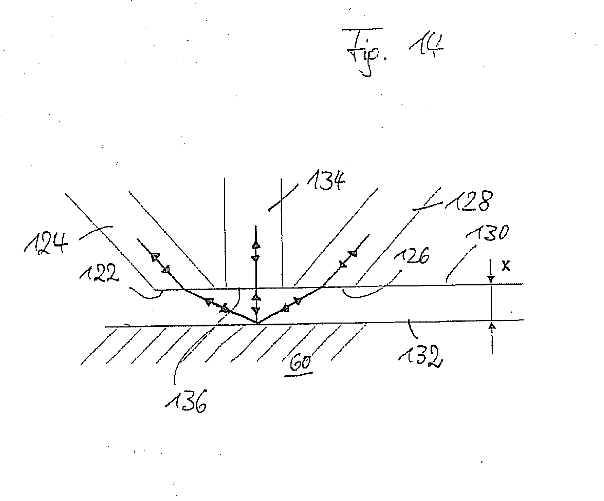

Fig. 14 zeigt eine Anordnung von drei Lichteiterfasern, wobei eine mittlere Faser 134 senkrecht zur Oberfläche 132 des Objekts 60 gerichtet ist und die Lichtleiter 124 und 128 an sich gegenüberliegenden Seiten des Lichtleiters 134 symmetrisch derart angeordnet sind, dass sich die Lichtbündel, die aus den Fenstern 122, 126 und 136 der Lichtleiter austreten bzw. in diese eintreten, in einem Punkt auf der Oberfläche 132 des Objekts 60 schneiden.

In allen drei Beispielen können die Lichtleiter, je nach deren Anschluss an die Strahlungsquellen, den Detektor oder die Kopplungseinrichtung, als Lichtleiter zum Senden der Referenzstrahlung, zum Empfangen der Referenzrückstrahlung, zum Senden der Anregungsstrahlung und/oder zum Empfangen der Anregungsrückstrahlung dienen.

Für die Analyse der Oberfläche 132 eines Objekts 60 kann als Anregungsrückstrahlung sowohl die reflektierte Anregungsstrahlung als auch von der Oberfläche in Folge von deren Beaufschlagung mit Anregungsstrahlung ausgehende Lumineszenzstrahlung erfasst werden. Die Sende- und Empfangsfasern können zentral (senkrecht auf der Oberfläche 132 stehend) oder unter einem Winkel zueinander angeordnet werden, wobei für die Referenzstrahlung und die Anregungsstrahlung gemeinsame Austrittsfenster und/oder Eintrittsfenster benutzt werden können (vergleiche Fig. 6). Die Endfläche eines einzigen, vorteilhafterweise senkrecht auf der zu untersuchenden Oberfläche stehenden Lichtleiters kann sowohl als Austrittsfenster als auch als Eintrittsfenster benutzt werden.

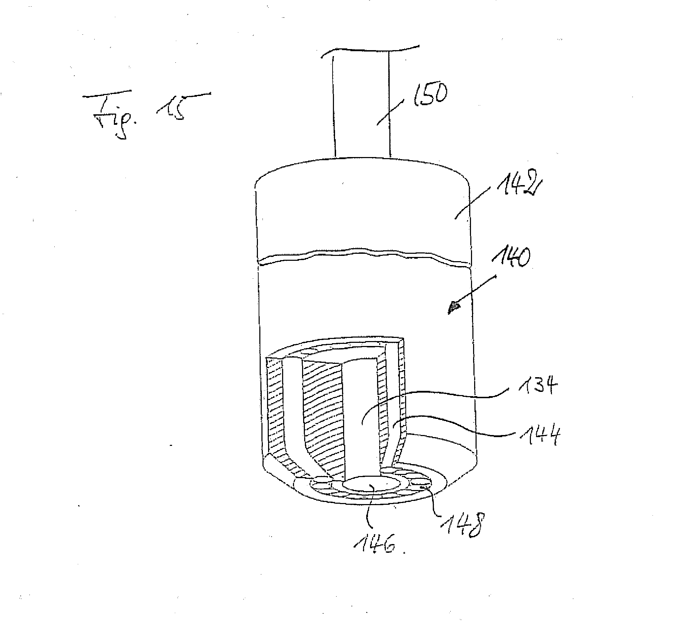

Die Öffnungen, durch die Referenz- und/oder Anregungsstrahlung austritt und Rückstrahlung eintritt, können unmittelbar als Löcher in einem Gehäuse ausgebildet sein, hinter denen entsprechende Optiken angeordnet sind, oder können als Austritts- und Eintrittsfenster von Glasfasern bzw. Lichtleitern ausgebildet sein, die in den jeweiligen Öffnungen enden. Vorteilhaft sind die Austrittsfenster und Eintrittsfenster der Lichtleiter in einem Sensorkopf 140 gemäß Fig. 15 angeordnet. In einem Gehäuse 142 des Sensorkopfes sind die einzelnen Lichtleiter 134 und 144 sicher aufgenommen und enden in Stirnflächen 146 bzw. 148, die vorteilhaft in einer gemeinsamen Ebene liegen. Die Lichtleiter sind durch das Gehäuse 142 hindurch geführt und zu einem flexiblen, gegebenenfalls ummantelten Lichtleiterbündel 150 zusammengefasst, dessen Lichtleiter mit jeweiligen Einrichtungen, wie Strahlungsquellen, Kopplungseinrichtung und Detektor verbunden sind.

Im dargestellten Beispiel sind Stirnflächen 148 kreisförmig um eine zentrale Stirnfläche 146 angeordnet, so dass die Anordnung gemäß Fig. 14 als ein senkrechter Schnitt durch das Vorderende des Sensorkopfes 140 betrachtet werden kann.

Die Zuordnung der einzelnen Lichtleiter zu den Strahlungsquellen und dem Detektor oder Detektoren richtet sich nach Zweckmäßigkeiten. So kann beispielsweise eine Stirnfläche 148 eines Lichtleiters 144 ein Referenzstrahlungsaustrittsfenster bilden und eine diametral gegenüberliegende Stirnfläche 148 kann ein Referenzrückstrahlungseintrittsfenster bilden. Es müssen nicht alle Lichtleiter aktiviert sein, so dass ein Sensorkopf für unterschiedlichste Anwendungen verwendet werden kann und an unterschiedlichste Einrichtungen angeschlossen werden kann.

Im Betrieb kann der kompakte und bewegbare Sensorkopf an das zu untersuchende Objekt herangeführt werden, wobei die Referenzrückstrahlung in Abhängigkeit vom Abstand vom Objekt ausgewertet wird und bei Vorliegen vorbestimmter Bedingungen, beispielsweise beim Maximum der Referenzrückstrahlung, die Anregungsstrahlung aktiviert wird, so dass die Anregungsrückstrahlung analysiert werden kann. Die Einhaltung eines ermittelten nominalen Abstandes kann durch Anbringen einer Abstandshülse auf dem Sensorkopf realisiert werden.

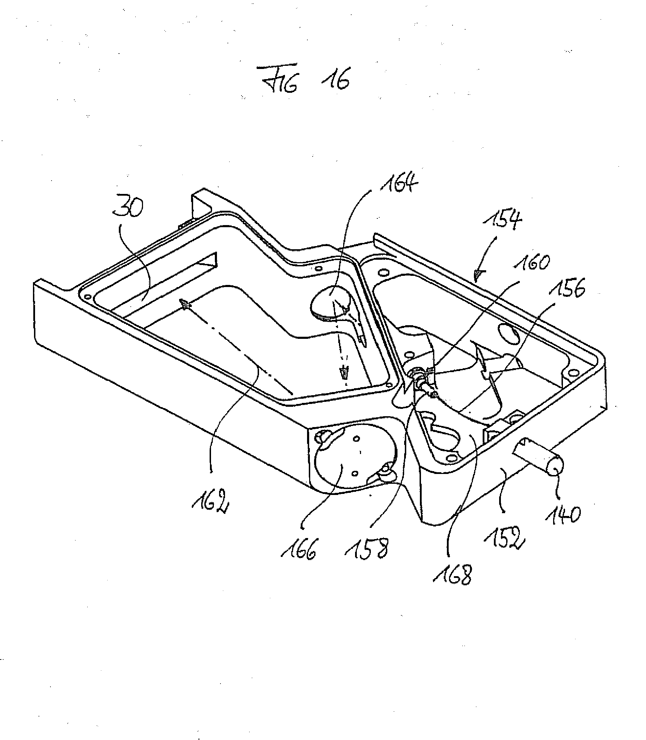

Fig. 16 zeigt ein Handspektrometer mit abgenommenem Gehäusedeckel. In eine Stirnwand 152 des Gehäuses 154 ist ein Sensorkopf 140 eingesetzt, dessen Empfängerlichtleiter 156, der beispielsweise die Referenzrückstrahlung und die Anregungsrückstrahlung aufnimmt, in einer Ferrule 158 einer Auskopplungseinrichtung 160 endet. Der zu analysierende Lichtstrahl 162 tritt aus der Auskopplungseinrichtung 160 in bezüglich der geometrischen Optik wohldefinierter Weise aus, wird an einem Spiegel 164 reflektiert, gelangt auf eine Spektralzerlegungseinheit 166, beispielsweise ein Beugungsgitter, und von dort auf den Detektor 30. Die Umlenkung des Lichtstrahls über den Spiegel 164 hat den Vorteil, dass die Stirnwand 152 relativ schmal ausgeführt werden kann, so dass das gesamte Gerät kompakt ist. Die Strahlungsquellen, Kopplungseinrichtungen, Auswerteelektronik, Energieversorgung usw. können in dem Fach 168 des Gehäuses 154 untergebracht sein.

Wie sich aus dem Vorstehenden ergibt, ist mit der beschriebenen Vorrichtung ein Verfahren zum sicheren Emitieren einer Anregungsstrahlung durchführbar, welches Verfahren folgende Schritte enthält: Emitieren einer Referenzstrahlung (18) zur Beaufschlagung eines mit der Referenzstrahlung (18) und der Anregungsstrahlung (14) beaufschlagbaren Objekts (60), das sich an einer vorbestimmten Position befindet, mit der Referenzstrahlung (18); Detektieren einer von dem Objekt (60) aufgrund der Beaufschlagung mit der Referenzstrahlung (18) erzeugten Referenzrückstrahlung (15); Erzeugen eines Detektionssignals basierend auf der detektierten Referenzrückstrahlung (15); Überprüfen, ob das Detektionssignal wenigstens eine vorbestimmte Bedingung erfüllt; und Beaufschlagung des sich an der vorbestimmten Position befindenden Objekts (60) mit der Anregungsstrahlung (14) nur dann, wenn die vorbestimmte Bedingung erfüllt ist.

Das Überprüfen des Detektionssignals kann ein Vergleichen eines in dem Detektionssignal enthaltenen Spektrums der Referenzstrahlung mit einem in einem der Referenzstrahlung zugeordneten Referenzsignal enthaltenen Referenzspektrum enthalten.

Eine vorbestimmte Bedingung kann darin bestehen, dass die Referenzrückstrahlung eine einen Schwellenwert übersteigende Intensität hat.

Die Referenzstrahlung kann aus einem Referenzstrahlungsaustrittsfenster austreten und die Referenzrückstrahlung kann in ein Referenzrückstrahlungseintrittsfenster eintreten und es kann überprüft werden, ob das Detektionssignal im Bereich eines Maximums einer Kurve liegt, die das Detektionssignal in Abhängigkeit von dem Abstand zwischen dem Austrittsfenster und/ oder dem Eintrittsfenster von der Oberfläche des Objekts angibt, und die Emission der Anregungsstrahlung kann nur dann ermöglicht werden, wenn das Detektionssignal im Bereich des Maximums liegt.

Bei einer anderen Durchführungsform des Verfahrens kann die Referenzstrahlung aus einem Referenzstrahlungsaustrittsfenster austreten und die Referenzrückstrahlung in ein Referenzrückstrahlungseintrittsfenster eintreten und überprüft werden, ob das Detektionssignal im mittleren Bereich einer aufsteigenden Flanke einer Kurve liegt, die das Detektionssignal in Abhängigkeit von dem Abstand zwischen dem Austrittsfenster und/oder dem Eintrittsfenster von der Oberfläche des Objekts angibt, und die Emission der Anregungsstrahlung kann nur dann ermöglicht werden, wenn das Detektionssignal im Bereich der aufsteigenden Flanke liegt.

Bezugszeichenliste:

10 Gehäuse

14 Anregungsstrahlung

15 Rückstrahlung

18 Referenzstrahlung

20 Anregungsstrahlungsquelle

30 Detektor

40 Datenverarbeitungseinheit

60 Objekt

70 Referenzstrahlungsquelle

90 Kopplungseinrichtung

100 Kopplungseinrichtung

110 Kopplungseinrichtung

120 Kopplungseinrichtung

122 Strahlungsaustrittsfenster

124 Lichtleiter

126 Strahlungseintrittsfenster

128 Lichtleiter

130 Fensterebene

132 Oberfläche

134 Lichtleiter

140 Sensorkopf

142 Gehäuse

144 Lichtleiter

146 Stirnfläche

148 Stirnfläche

150 Lichtleiterbündel

152 Stirnwand

154 Gehäuse

156 Empfängerlichtleiter

158 Ferrule

160 Auskopplungseinrichtung

162 Lichtstrahl

164 Spiegel

166 Spektralzerlegungseinheit

168 Fach

**Patentansprüche**

1. Vorrichtung zum sicheren Emittieren einer Anregungsstrahlung (14), enthaltend eine Anregungsstrahlungsquelle (20) zum Erzeugen einer aus einem Anregungsstrahlungsaustrittsfenster austretenden Anregungsstrahlung (14),
eine Referenzstrahlungsquelle (70) zum Erzeugen einer aus einem Referenzstrahlungsaustrittsfenster (122) austretenden Referenzstrahlung (18) zur Beaufschlagung eines mit der Referenzstrahlung (18) und der Anregungsstrahlung (14) beaufschlagbaren Objekts (60), das sich an einer vorbestimmten Position befindet, mit der Referenzstrahlung (18),
eine Detektoreinrichtung (30) zum Detektieren einer von dem Objekt (60) aufgrund der Beaufschlagung mit der Referenzstrahlung (18) erzeugten, durch ein Referenzrückstrahlungseintrittsfenster (126) eintretenden Referenzrückstrahlung (15) und Erzeugen eines Detektionssignals basierend auf der detektierten Referenzrückstrahlung (15),
eine Bestimmungseinrichtung (40) zum Überprüfen, ob das Detektionssignal wenigstens eine vorbestimmte Bedingung erfüllt, und
eine Sicherheitseinrichtung (40) zum Ermöglichen einer Beaufschlagung des sich an der vorbestimmten Position befindenden Objekts (60) mit der Anregungsstrahlung (14) durch die Anregungsstrahlungsquelle (20) nur dann, wenn die vorbestimmte Bedingung erfüllt ist.

2. Vorrichtung nach Anspruch 1, wobei das Referenzstrahlungsaustrittsfenster (122) und das Referenzrückstrahlungseintrittsfenster (126) einen vorbestimmten Abstand voneinander haben.

3. Vorrichtung nach Anspruch 2, **dadurch gekennzeichnet, dass** eine der der aus dem Referenzstrahlungsaustrittsfenster (122) austretenden Referenzstrahlung (18) entsprechende Gerade eine der der in das Referenzrückstrahlungseintrittsfenster (126) eintretenden und auf die Detektoreinrichtung fallenden Referenzrückstrahlung (15) entsprechende Gerade in einem Schnittpunkt schneidet.

4. Vorrichtung nach Anspruch 3, wobei eine Gerade, die der aus dem Anregungsstrahlungsaustrittsfenster austretenden Anregungsstrahlung (14) entspricht, durch den Schnittpunkt geht.

5. Vorrichtung nach Anspruch 4, wobei eine Gerade, die der in ein Anregungsrückstrahlungseintrittsfenster eintretenden und auf die Detektoreinrichtung fallenden Anregungsrückstrahlung (15) entspricht, durch den Schnittpunkt geht.

6. Vorrichtung nach einem der Ansprüche 1 bis 5, enthaltend eine Kopplungseinrichtung (90; 110; 120) zum Leiten eines Teils der Referenzstrahlung (18) zu der Detektoreinrichtung (30), wobei die Detektoreinrichtung (30) zum Erzeugen eines Referenzsignals und Weitergeben des Referenzsignals zu der Bestimmungseinrichtung (40) ausgebildet ist.

7. Vorrichtung nach einem der Ansprüche 1 bis 6, enthaltend mehrere Anregungsstrahlungsquellen (20A, 20B, 20C) und eine Kopplungseinrichtung (110; 120) zum Koppeln der von den mehreren Anregungsstrahlungsquellen (20A, 20B, 20C) erzeugten Anregungsstrahlung (14) und der Referenzstrahlung (18) derart, dass die Referenzstrahlung und die gekoppelte Anregungsstrahlung aus einem gemeinsamen Austrittsfenster austreten.

8. Vorrichtung nach Anspruch 7, wobei die Detektoreinrichtung (30) in die Kopplungseinrichtung (120) integriert ist und/oder die Kopplungseinrichtung (110) eine Spiegelanordnung (S1-S4) oder eine Prismenanordnung (S1-S4) zum Leiten eines ersten Teils der Referenzstrahlung (18) und der von den Anregungsstrahlungsquellen (20A, 20B, 20C) erzeugten Anregungsstrahlung (14) zu einem optischen Leiter (16) und Leiten eines zweiten Teils der Referenzstrahlung (18) und zumindest eines Teils der Referenzrückstrahlung (15) von dem optischen Leiter (16) zu der Detektoreinrichtung (30) enthält.

9. Vorrichtung nach einem der Ansprüche 1 bis 8, wobei die Referenzstrahlung (18) eine erste Polarisation aufweist und zwischen dem Referenzrückstrahlungseintrittsfenster (126) und der Detektoreinrichtung (30) ein Filter zum Herausfiltern der Referenzrückstrahlung (15) mit der ersten Polarisation angeordnet ist.

10. Vorrichtung nach einem der Ansprüche 1 bis 9, wobei die Vorrichtung als autarkes Handgerät ausgebildet ist.

11. Vorrichtung nach einem der Ansprüche 1 bis 10, wobei die Strahlungseintritts- und Strahlungsaustrittsfenster als Stirnflächen (146, 148) von Lichtleitern (134, 144) ausgebildet sind.

12. Vorrichtung nach Anspruch 11, wobei das Strahlungsaustrittsfenster und/oder das Strahlungseintrittsfenster wenigstens eines Lichtleiters (144) zu dessen Längsrichtung geneigt sind/ist.

13. Vorrichtung nach Anspruch 11 oder 12, wobei alle Strahlungseintritts- und Strahlungsaustrittsfenster in einer gemeinsamen Ebene (120) angeordnet sind.

14. Sensorkopf für eine Vorrichtung nach einem der Ansprüche 11 bis 13, enthaltend ein Sensorgehäuse (142), an dem alle Strahlungsaustritts- und Strahlungseintrittsfenster (146, 148) der Lichtleiter (134, 144) in einer Ebene liegend ausgebildet sind und der mit den Strahlungsquellen und der Detektoreinrichtung (30) über ein Bündel (150) der entsprechenden Lichtleiter verbindbar ist.

15. Verfahren zum sicheren Emittieren einer Anregungsstrahlung (14), enthaltend Emittieren einer Referenzstrahlung (18) zur Beaufschlagung eines mit der Referenzstrahlung (18) und der Anregungsstrahlung (14) beaufschlagbaren Objekts (60), das sich an einer vorbestimmten Position befindet, mit der Referenzstrahlung (18),
Detektieren einer von dem Objekt (60) aufgrund der Beaufschlagung mit der Referenzstrahlung (18) erzeugten Referenzrückstrahlung (15),
Erzeugen eines Detektionssignals basierend auf der detektierten Referenzrückstrahlung (15), Überprüfen, ob das Detektionssignal wenigstens eine vorbestimmte Bedingung erfüllt, und Beaufschlagung des sich an der vorbestimmten Position befindenden Objekts (60) mit der Anregungsstrahlung (14) nur dann, wenn die vorbestimmte Bedingung erfüllt ist.

16. Verfahren nach Anspruch 15, wobei das Überprüfen des Detektionssignals ein Vergleichen eines in dem Detektionssignal enthaltenen Spektrums der Referenzrückstrahlung (15) mit einem Referenzspektrum enthält, das in einem der Referenzstrahlung (18) zugeordneten Referenzsignal enthalten ist.

17. Verfahren nach Anspruch 15 oder 16, wobei das Überprüfen des Detektionssignals ein Überprüfen enthält, ob wenigstens eine der folgenden Bedingungen erfüllt ist:

   - die Referenzrückstrahlung (15) weist eine einen Schwellenwert übersteigende Intensität auf,
   - das Detektionssignal liegt in einem Bereich eines Maximums einer Kurve, die das Detektionssignal in Abhängigkeit von dem Abstand zwischen einem Austrittsfenster der Referenzstrahlung (18) und/oder einem Eintrittsfenster der Referenzrückstrahlung (15) und dem Objekt (60) angibt,
   - das Detektionssignal liegt in einem mittleren Bereich einer aufsteigenden Flanke einer Kurve, die das Detektionssignal in Abhängigkeit von dem Abstand zwischen einem Austrittsfenster der Referenzstrahlung (18) und/oder einem Eintrittsfenster der Referenzrückstrahlung (15) und dem Objekt (60) angibt.

Fig. 1

Fig. 2

EP 2 345 889 A2

Fig.3

Fig. 4

12

Fig. 5

Fig. 6

Fig. 7

Fig. 8

Fig. 9

20B

S4

S2

30

70

S3

S1

20A

13

Fig. 10

Fig. 11

Fig. 12

Fig. 13

Fig. 14

Fig. 15

FIG 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006032701 B3
- US 20040109534 A1
- DE 60105386 T2
- US 20060092415 A1